(19) Europäisches Patentamt / European Patent Office / Office européen des brevets

(11) **EP 3 824 799 A1**

(12) **EUROPEAN PATENT APPLICATION**

(43) Date of publication:
26.05.2021 Bulletin 2021/21

(21) Application number: 19210464.4

(22) Date of filing: 20.11.2019

(51) Int Cl.:
A61B 5/00 (2006.01)　　　　A61B 1/00 (2006.01)
A61B 1/04 (2006.01)　　　　G01N 21/47 (2006.01)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
**KH MA MD TN**

(71) Applicant: Helmholtz Zentrum München - Deutsches
Forschungszentrum für Gesundheit und Umwelt (GmbH)
85764 Neuherberg (DE)

(72) Inventors:
• KOCH, Maximilian
  81679 München (DE)
• Ntziachristos, Vasilis
  80939 München (DE)

(74) Representative: Linsmeier, Josef
Wallinger Ricker Schlotter Tostmann
Patent- und Rechtsanwälte Partnerschaft mbB
Zweibrückenstrasse 5-7
80331 München (DE)

(54) **DEVICE, APPARATUS AND METHOD FOR IMAGING AN OBJECT**

(57) The invention relates to a device, an apparatus and corresponding method for imaging an object, in particular a biological tissue, wherein the device comprises: at least one illumination unit (140, 145) configured to illuminate a region of interest (100) of an object with homogeneous illumination light configured to stimulate the region of interest (100) of the object to emit emission light, in particular fluorescence light, and patterned illumination light exhibiting a spatially varying light intensity over the region of interest (100) of the object; at least one image capturing unit (110, 120) configured to capture at least one marker light image by detecting the emission light emitted by the region of interest (100) and at least one sample light image by detecting light reflected, in particular diffusely reflected, by the region of interest (100) upon illumination with the patterned illumination light; and a processing unit configured to generate at least one corrected marker light image based on the at least one marker light image and the at least one sample light image.

Fig. 1

**Description**

The present invention relates to a device, an apparatus and a corresponding method for imaging an object, in particular a biological tissue, according to the independent claims.

Optical imaging is widely employed in medicine during endoscopic procedures, in surgery, ophthalmology, intervention and other applications. Healthy and diseased tissues exhibit differences in several properties, such as structural, compositional, metabolic, molecular and cellular properties, that can be detected, e.g., by a camera. Detection is based either in intrinsic tissue chromophores such as hemoglobin or melanin or extrinsically administered agents (e.g. fluorochromes) which can in-vivo stain physiological, cellular or molecular features (e.g. perfusion, permeability, inflammation, receptor distribution etc). Local or systemic administration of agents with specificity to cellular and subcellular tissue and disease biomarkers can alter the optical properties of healthy and diseased tissue in a different way resulting in visualization of lesions with high contrast with the background healthy tissues. Recent studies indicate that the use of externally administered fluorescent agents is a highly promising approach since fluorescence signals can provide high contrast. For example, engineered agents can be very sensitive and specific in cancer detection by targeting specific molecular features of carcinogenesis and tumor lesions.

It is an objective of present invention to provide a device, an apparatus and a corresponding method for improved imaging of an object, in particular a biological tissue. In particular, an objective of the invention is to provide an imaging device, in particular for multi-parametric real-time medical imaging, being capable of avoiding disadvantages of conventional techniques. Furthermore, the objective of the invention is to provide an imaging method, in particular for collecting and providing accurate photonic images for biomedical imaging, being capable of avoiding the disadvantages of conventional techniques.

The above objective is achieved by a device, an apparatus and a method according to the independent claims. Preferred embodiments are defined in the dependent claims.

A device for imaging an object, in particular a biological tissue, according to a first aspect of the invention comprises: at least one illumination unit configured to illuminate a region of interest of an object with homogeneous illumination light configured to stimulate the region of interest of the object to emit emission light, in particular fluorescence light, and with patterned illumination light exhibiting a spatially varying light intensity over the region of interest; at least one image capturing unit configured to capture at least one marker light image by detecting the emission light emitted by the region of interest of the object and at least one sample light image by detecting light reflected, in particular diffusely reflected, by the region of interest upon illumination with the patterned illumination light; and a processing unit configured to generate at least one corrected marker light image based on the at least one marker light image and the at least one sample light image.

A method for imaging an object, in particular a biological tissue, according to a second aspect of the invention comprises the following steps: illuminating a region of interest of an object with homogeneous illumination light to stimulate the region of interest of the object to emit emission light, in particular fluorescence light; illuminating the region of interest of the object with patterned illumination light exhibiting a spatially varying light intensity over the region of interest; capturing at least one marker light image by detecting the emission light emitted by the region of interest of the object; capturing at least one sample light image by detecting light reflected, in particular diffusely reflected, by the region of interest upon illumination with the patterned illumination light; generating at least one corrected marker light image based on the at least one marker light image and the at least one sample light image.

An apparatus for determining and visualizing optical properties of biological tissue, in particular for use in a device according to the first aspect if the invention and/or in a method according to the second aspect of the invention, comprises: an illumination unit configured to illuminate a region of interest of an object, in particular a biological tissue, with patterned illumination light exhibiting a spatially varying light intensity over the region of interest; an image capturing unit configured to capture at least one sample light image of the region of interest by detecting light reflected, in particular diffusely reflected, by the region of interest of the object upon illumination with the patterned illumination light; and a processing unit configured to generate, based on the at least one sample light image, at least one image visualizing the at least one optical property of the object, in particular light absorption and/or light scattering coefficients.

An apparatus for determining correction data, in particular for use in or with a device according to the first aspect and/or in a method according to the second aspect of the invention, comprises: an illumination unit configured to illuminate a region of interest of an object, in particular a biological tissue, with patterned illumination light exhibiting a spatially varying light intensity over the region of interest; an image capturing unit configured to capture at least one sample light image of the region of interest by detecting light reflected, in particular diffusely reflected, by the region of interest of the object upon illumination with the patterned illumination light; a processing unit configured to generate, based on the at least one sample light image, correction data for correcting at least one marker light image, which is obtainable by detecting emission light emitted by the region of interest upon illumination with homogeneous illumination light, the correction data relating to at least one optical property of the object, in particular light absorption and/or light scattering coefficients, and/or to at least one spatially varying total point spread function (tpsf).

Aspects of the invention are preferably based on the approach of capturing a spatially-dependent response, also referred to as "sample light image", of a tissue under test corresponding to light reflected by the tissue in response to a patterned illumination and utilizing information contained in the captured response to correct a marker light image of the tissue, which is obtained from light, in particular fluorescence light, emitted by the tissue in response to illuminating the tissue with homogeneous illumination light, so as to obtain at least one corrected marker light image.

The spatially-dependent response, i.e. the sample light image, of the tissue contains information regarding optical properties of the tissue, e.g. photon scattering and/or absorption by the tissue, having a possibly adverse impact on the captured marker light images, preferably fluorescence images. Thus, by using the sample light image of the tissue and/or at least a part of the information contained in the sample light image of the tissue for correcting marker light images, the influence of optical properties of the tissue on the captured marker light images can be reduced or even eliminated.

As a result, corrected marker light images obtained in this way contain more accurate and/or quantitative information, in particular regarding a spatial distribution of fluorophores and/or chromophores in the tissue. Further, possible artifacts in the marker light images are reduced or even eliminated.

In summary, the invention provides a device, an apparatus and a corresponding method for improved imaging of an object, in particular a biological tissue.

Within present disclosure, the homogeneous illumination light is also referred to as "broad illumination", "non-patterned illumination", "excitation light", "excitation illumination" or "planar wave excitation", and the patterned illumination light is also referred to as "inhomogeneous illumination" or "sample light pattern". Further, emission light is also referred to as "marker light". Further, the sample light image is also referred to as "patterned light image". Further, unless otherwise stated, the terms "object", "tissue" and "sample" are used synonymously. Further, the region of interest is also referred to as "region under investigation".

Preferably, the device comprises at least one illumination unit being arranged for illumination of a sample under investigation with illumination light and one or more image capturing units, also referred to as "detectors", being arranged for collecting different images of the sample.

Preferably, the illumination unit comprises at least one broad illumination arrangement and at least one pattern illumination arrangement. Preferably, broad illumination refers to light of constant intensity over an area, in particular the region of interest. Preferably, pattern illumination refers to light of varying intensity over an area, in particular the region of interest. The detectors preferably include at least one sample light camera being capable of sensing sample light coming from the sample in a spatially resolved manner. Preferably, the cameras can be optical cameras, i.e. a Charged Coupled Device (CCD), Complementary metal-oxide-semiconductor (CMOS), Indium gallium arsenide based detector (InGaAs), or any other sensor device, known in the art, capable to quantize optical radiation.

Preferably, broad and pattern illumination of the sample under investigation can occur simultaneously or at different time points. For simultaneous illumination the two illumination fields preferably superimpose. In case of simultaneous illumination, a differentiation regarding image capturing, i.e. whether the sample light image or marker light image is captured, can preferably occur using spectral separation or spatial separation, using corresponding filtering operations, as further elucidated in the preferred embodiments below.

Preferably, the processing unit is further configured to extract optical property information regarding at least one optical property, in particular light absorption and/or light scattering coefficients, of the object from the at least one sample light image and to generate the at least one corrected marker light image by correcting the at least one marker light image using the optical property information. In this way, spatially dependent optical, in particular absorption and/or scattering, properties of the tissue can be explicitly determined and/or decomposed and/or extracted from the sample light image and used for correcting the at least one marker light image.

Preferably, the processing unit is further configured to determine at least one spatially varying total point spread function (tpsf) from the at least one sample light image and to determine the optical property information from one or more properties, in particular functional parameters, of the total point spread function (tpsf) determined from the at least one sample light image. This allows for a particularly reliable determination of, e.g., scattering and/or absorption properties of the tissue and an accordingly reliable correction of the marker light image.

Alternatively or additionally, the processing unit is preferably configured to determine at least one spatially varying total point spread function (tpsf) from the at least one sample light image and to generate the at least one corrected marker light image by deconvolving the at least one marker light image using the at least one spatially varying total point spread function (tpsf) determined from the at least one sample light image. By correcting the marker light image using the tpsf, the optical properties of the tissue are considered "implicitly", because the shape and/or height of the tpsf is implicitly determined by the optical properties of the tissue, e.g. absorption and/or scattering coefficients, whereas an explicit determination of absorption and/or scattering coefficients of the tissue, e.g. in an intermediate processing step, is dispensable. Further, deconvolving the at least one marker light image using the at least one spatially varying total point spread function (tpsf) allows for a particularly exact way to reduce or eliminate the influence of the optical properties of the tissue on the marker light image. Preferably, a point-patterned illumination is utilized for determining optimal parameters of a space-variant deconvolution of marker light images (i.e. fluorescence images) using one or more measured, and therefore "non-blind", point spread functions (PSFs).

Preferably, the processing unit is further configured to determine the at least one spatially varying total point spread function (tpsf) by fitting at least one function, in particular a Gaussian function and/or a model function describing a modification of a point illumination by absorption and scattering in diffusive media, to the at least one sample light image. This allows for determining the tpsf in a particularly reliable and simple way.

For example, fitting of the tpsf can include the use of a model function describing the modification of a point illumination by absorption and scattering in a diffusive media (i.e. the tissue). For example, solutions derived using transport theory and simplifications of transport theory including, for example, the telegrapher's equation or the diffusion equation can be used. Furthermore, the shape of the tpsf can be related to pre-calculated numeric simulations of the light-transport theory, such as the solutions of Monte-Carlo simulations. When such functions are employed, the tpsf fits can then lead to estimations of the absorption and scattering tissue coefficients.

Preferably, a point spread function describes a response of the imaged object to a point illumination and/or the imaged object's impulse response to a focused illumination.

Preferably, the homogeneous illumination light exhibits a spatially homogeneous light distribution over the region of interest and/or a constant intensity over the region of interest. Alternatively or additionally, the patterned illumination light exhibits a spatially inhomogeneous light distribution over the region of interest and/or a two-dimensional distribution, in particular a grid, of points of light. Preferably, the at least one illumination unit comprising a light source configured to emit light and a light modulator configured to spatially modulate the intensity of the light emitted by the light source so as to obtain the patterned illumination light. Preferably, the light modulator comprising one or more light reflecting elements configured to spatially selectively reflect the light emitted by the light source so as to obtain the patterned illumination light and/or one or more light transmitting elements configured to spatially selectively transmit the light emitted by the light source so as to obtain the patterned illumination light. Each of the aforementioned embodiments allows for a particularly simple and robust generation of the patterned illumination light.

Preferably, the at least one image capturing unit comprises a marker light image camera configured to detect the emission light emitted by the region of interest of the object, a sample light image camera configured to detect the light reflected by the region of interest of the object upon illumination with the patterned illumination light, and a light splitting optics, in particular a beam splitter or dichroic mirror, configured to relay the emission light emitted by the region of interest of the object to the marker light image camera and to relay the light reflected by the region of interest of the object upon illumination with the patterned illumination light to the sample light image camera. In this way, at least a part of the imaging optics, e.g. a system of optical lenses or mirrors, can be commonly used for capturing both the marker light image and the sample light image.

Preferably, the light splitting optics being further configured to relay the patterned illumination light to the region of interest so as to illuminate the region of interest of the object. In this way, at least a part of the imaging optics can also be used for illuminating the object with the patterned illumination light.

In the following, further preferred or alternative aspects of the device, apparatus and/or methods are described.

Present disclosure preferably also relates to an imaging device, in particular for medical imaging, in particular, a photonic system for surface and sub-surface imaging of a sample. The device can be employed to image the spatial distribution of sample properties, including the distribution of a marker substance. Furthermore, present disclosure relates to an imaging method, in particular for quantitative tissue imaging, extracting morphological, physiological and molecular tissue parameters, using intrinsic or extrinsically administered chromophores or fluorochromes of sample optical properties. Preferred applications of the disclosure are present in medical imaging and in particular in creating diagnostic images or images used for guiding interventional procedures.

Advantageously, the illumination light can be designed in terms of geometric, temporal and/or spectral properties for adapting the illumination light to sample properties and image types to be collected. To this end, the illumination unit preferably includes at least one illumination light conditioning device adjusting at least one of a spectral characteristic, a temporal characteristic, a polarization, a direction and a light field shape of the illumination light. If the illumination unit includes multiple light sources, for each of them a specific illumination light conditioning device can be provided. Preferably, the illumination light conditioning device comprises at least one of a spectral filter, a polarization filter, an illumination optic and a bundle of optical fibers. In addition, the patterned illumination can be designed to illuminate tissue with a predetermined pattern, such as a grid of points.

Preferably, images generated by the patterned illumination reveal different sample features compared to the features generated by the broad illumination. To separate images generated by the patterned illumination over images generated by the broad illumination, the device can employ time-separation techniques, spectral separation techniques, pattern separation techniques or other techniques as elaborated at the preferred embodiments of the implementation. Furthermore, the imaging device preferably includes a processor device being adapted for separating and processing images of the sample generated due to the broad illumination (marker light images) and the patterned illumination (sample light images). At least one of the images separated is then employed by the processor to extract information on the properties of the sample. This information is then employed to generate at least one synthetic and/or corrected image, in particular corrected marker light image.

Preferably, the sample is a biological object, in particular, a human or animal body or a part thereof. In particular, the sample comprises biological tissue or a part thereof. Accordingly, the invention is preferably used for medical imaging.

According to a further advantageous embodiment, the device includes a light splitting imaging optic which is configured for imaging light from the sample onto more than one detector cameras, i.e. one or more image capturing units. This configuration can be employed to collect different images corresponding to additional tissue properties, including polarization, autofluorescence, or fluorescence emanating from markers administered to tissue for contrast enhancement. These images, also referred to herein as "marker images", are indicating of tissue intrinsic or extrinsic markers obtained in addition to the traditional color (reflectance) images obtained by a sample camera. Additional cameras, however, could be also employed to separate images in response to broad illumination vs patterned illumination as described in more detail below.

Preferably, the sample light may be relayed onto at least one sample light camera, while the marker light is relayed onto at least one marker light camera. Preferably, both the sample light image and the marker light image are collected simultaneously, thus allowing real-time processing of the multiple different images of the sample.

Preferably, the term "camera" may relate to various kinds of physical cameras, e.g. charged coupled devices (CCD), Complementary metal-oxide-semiconductor (CMOS), Indium gallium arsenide based detectors (InGaAs), or any other sensor device, known in the art, capable to detect, in a spatially resolved manner, and quantize optical radiation.

Preferably, a multi-spectral implementation can be implemented as part of the same camera or the use of different areas of a single sensor registering the sample and marker light images. This allows for a flexible adaptation of the imaging device to the requirements of a practical application where different signals collected utilize different signal strengths and different dynamic ranges.

In a preferred embodiment, the sensitivity of one or more of the independently sensitive sensor areas is automatically adapted, through variable attenuation or amplification of the sample signals collected or the corresponding electrical signals generated.

Preferably, each of the at least two camera fields is provided with a field filter configured to adjust a spectral sensitivity range of the respective camera field.

If changeable field filters are provided, the flexibility can be further improved. Said field-filter change of field filters can be implemented in an automatic (e.g. motorized) or manual (e.g. end-user operated) implementation.

Further, any other optical spectrally tunable filter can be advantageous.

Furthermore, the imaging device includes a processor device being adapted for processing the broad illumination sample (marker light image), patterned illumination sample (sample light image) and possible further images in parallel and rendering at least one corrected image based on the at least one sample light image and the marker light image. Preferably, the at least one corrected image can be processed and rendered in real-time, i.e. with a delay after the image collection such that the delay is negligible in terms of human visual perception, typically corresponding to delays that are less than 50 milliseconds.

Providing at least one corrected image in real-time mode also may include providing an image sequence (video sequence) of corrected images. As an example, the processor device can be configured for generating a video sequence of the at least one sample light image, at least one of the marker light images, the at least one corrected image or a combination thereof.

Preferably, the sample light collected with the sample light camera due to illumination is the portion of the illumination light reflected by the surface and diffusively reflected by sub-surface layers of the sample. The sample light collected with the light camera due to patterned illumination contains information on the light-tissue interactions.

Preferably, two major characteristics of the tissue can be read from the images obtained due to patterned illumination. The first relates to the modification of the intensity of the pattern projected onto the tissue by the tissue optical properties. The second relates to the modification of the spatial characteristics of the pattern due to tissue optical properties. Both the intensity and pattern "readings" can be employed to evaluate tissue properties and relate to tissue characteristics. The intensity and pattern readings can be also employed to correct marker light images and improve their resolution and accuracy or quantification. A preferred illumination pattern is a pattern of points projected onto the tissue. Preferable is the utilizing of a rotational symmetric repeated element to prevent the introduction of any angular directionality, such as it occurs with strips or linear one-dimensional sinusoidal waves. This pattern effectively samples the modification of the point due to tissue characteristics. Each point will be then visualized by the patterned light sample image as a broadened function possibly with modified intensity.

Preferably, the above described measurement allows for measuring and using a spatially varying total point spread function (tpsf), i.e. a point spread function (psf) that contains information on the tissue itself. Said patterned light images (sample light images) are acquired in a spectral range, at least in parts covering the spectral range of the projected illumination pattern. The measurement of the tpsf intensity and broadening can be performed as an absolute measurement or as a relative measurement. The latter using, for example, a reference medium with known properties in order to calibrate the pattern projected onto the tissue.

The reference medium can, for example, be a block with homogenous optical properties, for example, a resin phantom or a fluid phantom containing known amounts of scatter and absorber.

Intensity and broadening can be determined by fitting functions to the pattern observed in the sample light images, for example fitting a Gaussian function and extracting an intensity value and a broadness value. The fit can look at directional broadness, i.e. calculate the broadness value along different directions.

Preferably, quantification of scattering can be correlated with the higher cellular density relating to disease such as cancer, while quantification of absorption can be linked to hemodynamics, tissue oxygenation, hypoxia, hemoglobin concentration or perfusion. These readings become even more specific and accurate when multiple images are acquired at multiple wavelengths. This can preferably be achieved by utilizing illumination at multiple wavelengths and/or detection at a single or multiple spectral bands. In other words, spectral responses can be separated only by multi-wavelength illumination, only by multi-band detection or both. Multi-wavelength illumination can be typically achieved by illumination at distinct, discrete wavelengths or discrete narrow wavelength bands. Multi-band detection can be achieved by single or multiple cameras, using, for example, band-pass filters and/or dichroic mirrors to separate the different spectral regions. The cameras can detect different spectral regions through at least one light splitting optic so that different bands are collected simultaneously. Alternatively, a spectral collection can be obtained in a time-shared function by exchanging filters or using a filter that changes its spectral acceptance band.

Alternatively, a spectral collection can be obtained by time-sharing the activation of multiple illumination sources in time-synchronized subsequent full or partial image readouts. Alternatively, spectral decomposition systems, such as prisms, monochromators etc. can be employed.

Preferably, when using a light point illumination pattern, the spacing between the illuminated points and/or the wavelength of the illuminated points can be predefined and/or selected. In principle, different combinations of wavelength and spacing allow for determining and/or considering different tissue properties with the correction of the marker light images. For example, white light spots allow for a determination of tissue properties at all possible wavelengths, since a spectral camera can then preferentially select tpsf's at specific spectral bands. Alternatively an illumination unit, containing specific illumination lines or bands can be selected, for example a combination of a white light source and lasers emitting in the near-infrared (e.g. 650 nm 700 nm 750 nm, 800 nm, 850 nm), a combination of distinct illumination lines both in the visible and near-infrared (e.g. 450 nm 500 nm 600 nm 700 nm 800 nm). A simplified pattern can then be at a single wavelength tuned to the spectral band of interest.

A preferred implementation of the disclosed device and method is in improving the contrast and the resolution in fluorescence images in the near-infrared. In this case, a single wavelength close to the operating band of the fluorochrome (marker images) could be employed. An alternative implementation of the disclosed device and method is the extraction of oxygenated and deoxygenated hemoglobin (marker images). In this case, it is implicit that the device will operate by collecting sample images at multiple wavelengths. In this case, a projected pattern in the corresponding spectral bands can be employed to correct each image with the particular tpsf of the tissue in the corresponding wavelength. Preferably, the broadening of the pattern projected onto tissue may have another effect on the captured patterned light image (sample light image). Depending on the wavelength employed and the spacing used, tpsf's can start overlapping. Therefore instead of distinct broadened tpsf's, a waving function will be instead captured. This is a preferred implementation in order to increase the resolution by which the effect of tissue on the measurements can be measured. This waving function is two dimensional, i.e. it can reveal multi-directional information along all 360-degree directions. Said multi-directionality is particularly advantageous. While very dense spacing will eventually create a homogenous function, a preferred distance is one that allows waving whereby the lower intensity point of the waving function is no less than 1 % of the maximum intensity point of the function or 10% for the maximum intensity point of the function.

The utilization of structured illumination in at least two disjoint spectral bands allows for the separation of tpsf's that are closer together, than their advantageous waiving function may allow.

Preferably, the spectral separation can be achieved by (i) utilizing additional image-sensors to split off different bands, (ii) temporally aligned switching and tuning of filters, or (iii) a combination of thereof.

An advantageous multi-spectral illumination pattern is to maximize the minimum Euclidean distances between spectrally aligned illumination points. A problem classically referred to as "circle stacking".

Advantageously, present disclosure provides a method and a device for photonic medical imaging of marker substance which can through real-time collection and processing of multispectral data offer accurate, quantitative imaging of surface and subsurface tissues. In order to collect the sample and marker light images simultaneously, the imaging device preferably includes a light splitting imaging optics which is configured for imaging light from the sample onto the detector cameras. The sample light is relayed onto the at least one sample light camera, while the marker light is relayed onto the at least one marker light camera. In this case, both the sample light and the marker light images are collected simultaneously, thus allowing real-time processing of the multiple different images of the sample. Herein marker light indicates light associated with excitation and emission light to and from, respectively, the marker substance.

Preferably, sample light refers to light in the same waveband that is used to project the sample light pattern and light in the same waveband that is acquired after diffusive reflection of the tissue under test by the sample-light camera.

The preferred real-time device and method are particularly suitable for molecular imaging as they allow for associating the specific and accurate visualization of administered marker substances with specificity to certain healthy and diseased tissue marker substances. In this case, the marker light images are improved using the parameters obtained by the patterned light image and/or the broad light image. Improvement, in this case, implies the use of the information collected by the patterned light image to improve the resolution, accuracy, fidelity, quantification of the marker image.

The performance of fluorescence images is affected by tissues and an advantage of the disclosed device and method is to reverse the effects of tissues on the fluorescence signals. In particular, light diffusion and changes in the absorption and scatter by different tissues can alter the appearance of fluorochromes emitted from tissues, e.g. by blurring the image and altering its intensity. Preferably, by using tpsf fits, these effects can be captured. Preferably, by using correction algorithms, accurate fluorescence images can be restored. Preferably, a generalized restoration approach is a deconvolution, using spatially varying tpsf's measured and/or approximated for the tissue imaged.

Usually, an approach of applying deconvolution comprises using a single psf function, representative of the measurement system. Then the effects of the measurement system on the measurement can be reverted. Instead, according to preferred aspects of present disclosure a spatially varying psf is used, which models the effects of the tissue on the image and consequently allows for reverting these effects. The resulting image will have better resolution, be more accurate and quantitative and allow for a better delineation of fluorescence lesions, due to reducing the blurring.

For a preferred application in medical imaging, the disclosure can provide three steps of administration of one or more contrast agents or probes (marker substances), e.g. molecular probes, multispectral optical or optoacoustic imaging, and processing of captured images for real-time display of corrected information. The administration step is provided as an optional feature of the inventive method. It can be omitted if the sample already includes the at least one marker substance for natural reasons or due to previous treatment. The at least one corrected image obtained with the inventive imaging method is also called diagnostic image. The term "diagnostic image" refers to the fact, that the image can be used for finding a diagnosis, e. g. by a physician and/or by a subsequent image evaluation or identify with high specificity a problematic or suspicious lesion to lead to efficient guidance and intervention, for example, an intervention with therapeutic intent. As an example, the diagnostic image may include a map of the sample highlighting various sample conditions. Similarly, the diagnostic image can be used to guide surgical intervention or endoscopically administered biopsies and other surgical procedures. However, the diagnostic image as such does not deliver the diagnosis.

Preferably, the term "marker substance" refers to any molecule which can alter the marker light (emission light) so as to generate contrast. A common example is a fluorochrome which stains perfusion, permeability or specifically binds to a certain target in the sample, like target tissue, target cells or certain cell components, like proteins, and which exhibits an interaction with light (UV, VIS and/or IR wavelength ranges) resulting in a specific absorption and/or fluorescence. The concept of use of a marker substance is to highlight one or more tissue characteristics which are altered at the presence of a disease. The marker substance is also referred to as "biomarker", "probe" or "contrast agent". It is selected by the skilled person in dependence on the binding properties and the spectral properties thereof. In particular, the marker substance is selected such it targets and reveals a molecular, structural, functional or compositional feature of the tissue which specifically changes in a gradual manner during the disease progression. The presence of the marker substance preferably alters the optical properties of the tissue, e.g. fluorescence or absorbance, in a way that the detected optical signal could even reveal the progress of the disease. The sample includes one or more marker substances. If multiple different marker substances are provided, they preferably have different spectroscopic properties. Besides fluorochromes, the marker substances could be absorbing dyes, nanoparticles, polarization shifting moieties, Fluorescence Resonance Energy transfer molecules, Raman particles etc.

A disease like cancer is known to cause several tissue alterations and the probes used as a marker substance are in general designed to highlight one of those alterations, e.g. metabolic activity or receptor upregulation. Nevertheless, diseases like cancer do not always express the same characteristic changes and therefore, the probes inherently exhibit low sensitivity. Inversely, in other cases, some non-diseased tissues might also mimic one of the disease characteristics reducing thereby the probe specificity. Besides cancer, interventions associated with vascular, intravascular, neuronal, cardiac, reconstructive and other diseases and indications are also considered.

With the available specific and sensitive marker substances such as molecular probes, it is possible to provide excellent overall performance and clinical outcome using the method. Moreover, the combined use of multiple probes may be preferred in terms of further increasing the information obtained for decision making during guidance and intervention or the diagnostic ability since the detection of a disease can then be based on several features that characterize the disease rather than a single feature. Additionally, the combined use of multiple probes and other chromophoric agents such as non-specific fluorescence dyes could be utilized to correct for potential inhomogeneities in local blood perfusion which could otherwise affect the local delivery of the probes and thereby introduce biased measurements.

Advantageously, the disclosed device and method can be conducted with various types of images. According to preferred embodiments, the detector cameras are adapted for collecting at least two image types of a color image, a fluorescence image, a reflectance image, a patterned-illumination image and/or an excitation image. Preferably, each of the detector cameras is provided with a camera filter, which is adapted to the image type to be collected.

Preferably, the marker light (emission light) is light generated in response to the illumination of at least one marker substance. Intensity, spectral composition and geometric distribution of the marker light is determined by the specific interaction of the illumination light (excitation light, homogeneous illumination light) with the at the least one marker substance and the distribution thereof in the sample.

Preferably, the processor of the disclosed device provides the capability of real-time imaging the sample, wherein the images produced in real time are corrected images that account, i.a., for features that can lead to artifacts (i.e. false positive or false negative readings) that may be present in the raw images. Preferably, the processor includes at least one of Field Programmable Gate Arrays and Graphic Processing Units. As an advantage, those types of processors are commercially available at relatively low prices and provide the ideal solution for dedicated real-time data processing.

Optionally, a control device can be provided additionally to the processor. The control device can be configured for at least one of the following functions. Firstly, it can control the light source device (illumination unit), the detector cameras and/or the processor. Secondly, the control device can be provided with a display device displaying the at least one sample light image, at least one of the marker light images, the at least one corrected image.

Preferably, the terms "correction", "correcting" or "to correct" refer to changing the intensity in the pixels of the captured image (marker light image) to be corrected and subsequently projected, so that the resulting corrected image more accurately reflects the actual marker bio-distribution in the field of view of the image. A correction, therefore, may contain any of steps that improve the intensity in each image in view of 1) the effects of contamination of marker signals with endogenous tissue signals, such as auto-fluorescence signals, 2) the effects that the depth of the marker location has on the marker signal collects, and 3) the effect that the tissue optical properties have on the collected marker signal. For example, the effect that a marker fluorochrome is embedded in a highly scattering tissue volume and the associated signal suffers increase diffusion in the acquired raw image (marker image).

Although artifact-free fluorescence and reflectance images can accurately provide the spatial distribution of fluorescence probes and tissue chromophores, it is not always straight forward to conclude if a tissue area is diseased or not. The diagnostic capability of each diagnostic probe is based on targeting a characteristic difference of the disease compared to healthy tissue. Nevertheless, diseases like cancer exhibit a remarkable variability in their characteristics and therefore, a single probe targeting a single cancer feature is often not very specific. By combined evaluation of multiple cancer features by means of multiple probes, can provide significantly increased specificity and sensitivity. Additionally, the automatic fusion of the diagnostic information with the color image of the tissue under examination provides a convenient and easy to use modality, suitable for clinical use.

Preferred imaging systems, devices and corresponding methods may comprise a reflection imaging modality, a subsystem for spatial light modulation of an illumination reflection light, a subsystem for planar wave fluorescence light excitation, a subsystem for fluorescence light image relay and acquisition, an image-acquisition- and processing-subsystem configured in a way to (i) project a spatially modulated reflection pattern onto the tissue under test, (ii) acquire the reflected reflection image, (iii) extract tissue optical properties, such as the spatially varying point spread function (PSF, tpsf) of the tissue under test, (iv) illuminate the tissue under test with fluorescence excitation light in a planar wave front and acquiring the corresponding fluorescence image, and in a final step (v) correct said fluorescence image with correction-algorithms allowing to incorporate spatially varying information of the PSF.

Preferably, in addition a multi-spectral reflection image (e.g. tri-spectral, RGB) is acquired, in a time-shared or simultaneously in an optical-spectrum-shared implementation.

Preferably, the image acquisition and correction are implemented in short latency, specifically with latency less than 100 ms, preferably, less than 25 ms, to allow for real-time application and manipulation during medical interventions, such as surgery.

Preferably, reflection images (sample light images) and fluorescence images (marker light images) are performed through the same image relay lens and split spectrally to their associated imaging sensors.

Preferably, the correction algorithm is a spatially variant deconvolution algorithm.

Preferably, the correction algorithm is a machine learning algorithm, such as a convolutional neural network, previously trained to gold-standard results e.g. obtained by Monte-Carlo simulation or analytical methods.

Preferably, the correction algorithm is a localized inverse-problem solution, obtained by solving an analytical model of light-tissue interaction.

Preferably, the spatial light modulator for projecting the reflection pattern comprises a reflective light modulator, (e.g. micro-mirror device, scanning galvano-metric mirrors) or a transmissive light-modulation device (e.g. Liquid crystal display) or any other light modulation device, know to the field.

Preferably, the extracted parameters of the structured sample light images, in particular the parameters of the PSF, are used to calculate, estimate or or lookup the wavelength-dependent optical parameters regarding scattering and/or absorption.

Preferably, respective images of the optical parameters regarding scattering and absorption may be displayed to the user of the system, either in absolute or relative units.

Further advantages, features and examples of the present invention will be apparent from the following description of following figures:

Fig. 1    shows a schematic illustration of an example of an imaging device;

Fig. 2    shows a schematic illustration of an example of an image acquisition and processing workflow;

Fig. 3    (a) to (f) shows examples illustrating a preferred mode of operation of the device and according method steps;

Fig. 4    a) to f) visualizes an example of the concept of utilizing several wave-lengths to decrease the lowest distance for tpfs point;

Fig. 5    shows a schematic of a spatially adaptive impulse response correction (SAIRC) process to enhance fluorescence imaging;

Fig. 6    shows an example illustrating the SAIRC improvement in fluorescence image quality of a phantom;

Fig. 7    shows examples illustrating the SAIRC improvement in the quality of fluorescence images of a mouse with a tumor;

Fig. 8    shows examples illustrating SAIRC improvement in quality of fluorescence images of two mice with tumors; and

Fig. 9    shows metrics to estimate optimal parameters for the deconvolution algorithm.

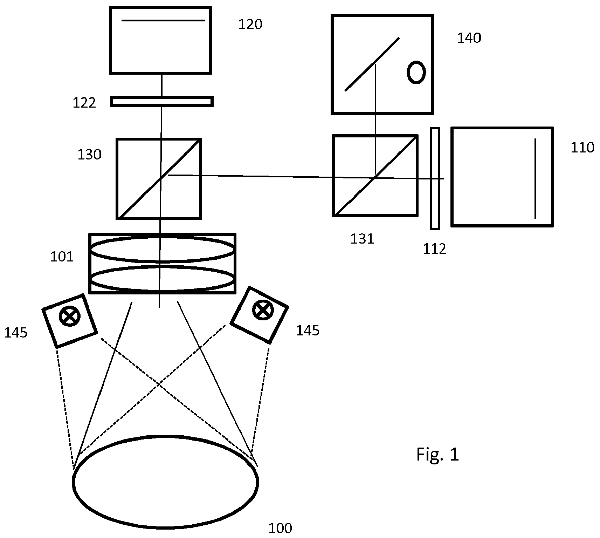

Figure 1 shows a schematic illustration of an example of an imaging device. A region under investigation 100 (e.g. a surgical cavity during a medical investigation) is relayed by a system of optical lenses 101 to at least one optical image sensor 120 for fluorescence measurements (e.g. a CCD, CMOS, InGaAs sensor).

The region under investigation 100 is illuminated by at least one, preferably homogeneous, excitation illumination 145 configured to induce fluorescence in a marker, in particular an applied marker, contained in the region 100.

An image guide path 101, 130, 122 to the fluorescence detector 120 is preferably equipped with an optical filter 122 to pass the desired fluorescence band, but reject or attenuate the wavebands of the excitation light or other wavebands. Additionally, the region under investigation 100 is relayed through imaging optics 101, a dichroic mirror or beamsplitter 130 onto a multispectral reflection and sample light camera 110.

Preferably, said imaging relay optics 101 is the same as for relaying the fluorescence path.

Preferably the beam-splitter or dichroic mirror 130 is configured to transmit the fluorescence emission waveband of the marker substance to the fluorescence sensor 120 and reflect all remaining wavebands.

Additionally, the optical output of a light shaping device 140 such as a digital micro mirror device (DMD)- or Liquid Crystal Display (LCD)-based projector is coupled to the same optical path by beam-splitters or dichotic mirrors 131, 130.

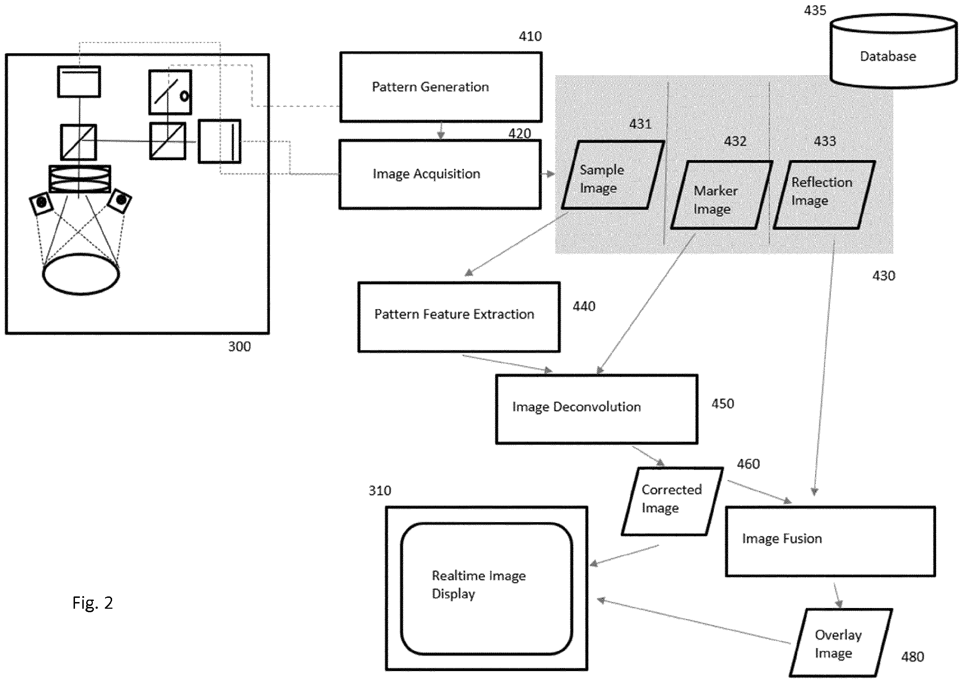

Figure 2 shows a schematic illustration of an example of an image acquisition and processing workflow, preferably using an imaging device 300 as shown in Figure 1.

The exemplarily illustrated implementation of the imaging device 300 comprises a sample pattern generation module 410 configured to generate a reflection sample pattern, e.g. the previously discussed point pattern. An image acquisition module 420 is configured to acquire the image response, i.e. a sample image 431, during illumination with the sample image illumination pattern. The image acquisition module 420 is further configured to acquire a marker image 432 (e.g fluorescence image). In a preferred implementation the marker image acquisition is performed in a time-parallel fashion.

Further, the image acquisition module 420 may also be configured to also acquire a multi-spectral reflection image 433 (e.g. Color RGB image). The image acquisition is preferably acquired by the same multispectral reflection sensor as the marker image. The multispectral reflection image and the marker image may be temporally or spectrally separated from each other.

The resulting image data 431, 432, 433 is stored in memory and may be saved in a persistent way for later analysis, documentation, training etc.

In a following step, a sample image feature extraction module 440 extracts features of the sample image 431.

In yet another following step an image deconvolution submodule 450 performs an image deconvolution of the marker-image 432 by applying the spatially variant tissue properties determined by the sample image feature extraction module 440. The resulting information is a corrected marker image 460 corrected by the modulating effect of biological tissue on light distribution, such as absorption and scattering.

In yet another step the corrected marker image 460 and the multispectral reflection image 433 may be fused, e.g. by image-alpha blending, to create a composite overlay image 480.

In a final step, the corrected marker image 460 and/or the composite overlay image 480 may be displayed to an end user by means of a real-time image display 310.

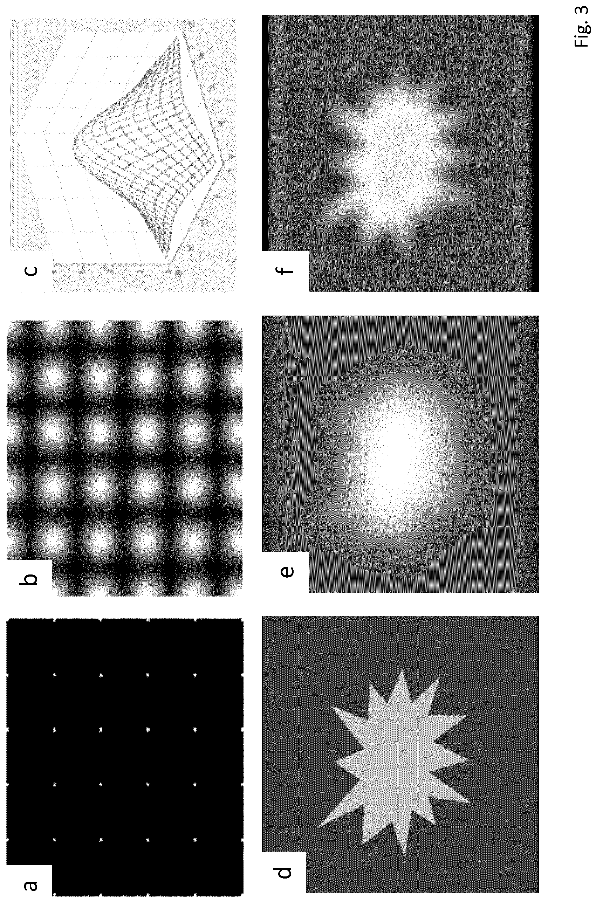

Figures 3 (a) to (f) show examples illustrating a preferred mode of operation of the device and according method steps for marker image correction using spatially variant point-spread-functions.

Figure 3 (a) shows a possible structured illumination pattern, projected onto the surface of the tissue under test. Figure 3 (b) shows a recorded image of a specific spectral reflection channel of different optical parameters. Figure (c) shows a 3-D intensity plot of a single fitted two-dimensional Gaussian of the form:

$$I = a*exp(-((x-x0).^2/2/sigmax^2 + (y-y0).^2/2/sigmay^2)) + b \qquad (1)$$

Where on the one hand the parameters a and b correlate on the absorption properties of the tissue under test, and on the other hand the parameters sigmax and sigmay depend mainly on the scattering parameters of the tissue under test.

Figure 3 (d) to (f) show examples of a complexly shaped fluorescence lesion. In Figure (d) the assumed true distribution of the fluorescence lesion is visualized (also referred to as "gold standard"). It can be seen that also the background shows a certain level of a fluorescence signal. Figure 3 (e) shows the captured fluorescence image (marker light image), as it is acquired using a planar wave fluorescence excitation without further image correction. Figure 3 (f) shows the corrected fluorescence image (corrected marker image) by deconvolution, using the spatially variant PSF parameters, as they were determined for every image region using Eq 1.

The shape and border of the fluorescence lesion has clearly resembled with improved accuracy in comparison to the uncorrected image in Figure 3 (e).

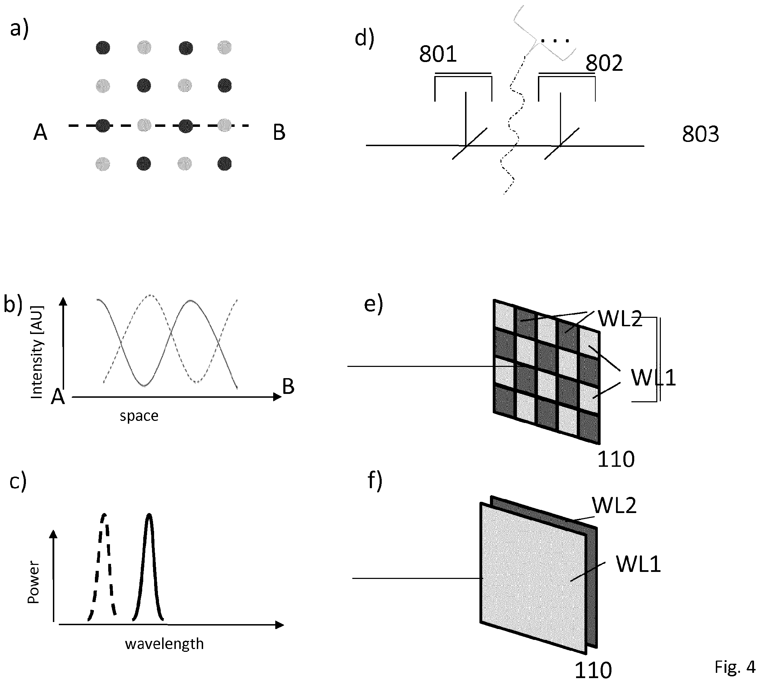

Figures 4 a) to f) visualize an example of the concept of utilizing several wavelengths to decrease the lowest distance for tpfs point, which remains feasible.

Figure 4 a) visualizes the distribution of points for different wavebands. This subfigure shows the distribution for two different wavebands for simplicity here (dark vs. bright spots). An optimal distribution varies with the number of disjoint wavebands.

Figure 4 b) shows a special profile for the resulting waving function after diffusive reflection along the profile line A - B from subfigure a). The solid and dashed line resembles the outcoupled optical intensity per spectrum.

Figure 4 c) visualizes the wavelength distribution of two mostly disjoint two wavebands.

Figure 4 d) shows one technical implementation in spectral-sharing the acquisition of the overlapping tpfs in combining dichroic mirrors and splitting the single wavebands of interest off to separated image sensors 801, 802, 803. In this mode of operation, the described subsystem in subfigure d) is to replace the part of the sample light camera 110 in the previously described implementations (see Figure 1).

Figure 4 e) shows yet another technical implementation in spectral-sharing the acquisition of overlapping tpsfs in the utilization of a sensor 110, equipped with a mosaic of spectrally different transmission filters, also known in the field as Bayer-patterned sensor. The spectral rejection and transmission properties should be designed to match the incident spectra, e.g. as shown in subfigure c)

Figure 4 f) shows yet another technical implementation, utilizing a multi-layer multi-spectral image sensor 110 acquiring the different spectral components in different sensor layers.

As the characterization and processing of the image is time-critical for intraoperative or interventional images, real-time processing, deconvolution, and quantification of the imaging data are particularly preferred for the clinical use of a diagnostic system. As real-time processing is quite demanding and conventional computer CPUs may have an insufficient performance, the acquired images are preferably separated into sub-problems capable of being solved on different worker processors. Such processors could be conventional Central Processing Units (CPUs) arranged in a compute-cluster, Field Programmable Gate Arrays (FPGAs), Graphics Processing Units (GPUs) or application-specific integrated circuits (ASICs)communicating over a low-latency data bus. Possible data bus technologies include (e.g. (Gig-Ethernet, optical fibers). The combination of the mentioned processors could be advantageous. The network-topologies could include star-, line- or more problem-oriented designs such as hierarchical or partly intermeshed topologies.

In the following, further preferred or alternative embodiments are discussed, in particular with reference to the optical set-up and data processing structure provided for obtaining multispectral photonic images in real time. Details of selecting suitable marker substances, preparing a sample, like introducing at least one marker substance type into the sample, designing the imaging optics, in particular with regard to focusing and magnification properties, operating the at least one light source, detector cameras and optionally further sensors and image processing techniques are not described here as far as they are known from prior art, in particular from conventional photonic systems for medical imaging. Furthermore, the imaging devices presented in the figures can be implemented in different ways depending on the specific needs of an application.

Preferably, the use of polarizers at the illumination and imaging optical paths can reduce and/or minimize the effects of specular reflection.

Preferably, the extraction of optical properties by mapping of single point-spread-functions is implemented in a parallelized fashion.

Preferably, the imaging system of Figure 1 facilitates an optical construction that splits the light collected by the imaging optics 101 into multiple imaging channels by means of dichroic mirrors or beam splitters 130, 131, and the associated optical filters 112, 122. The light splitting is preferably performed in such a way that each of the imaging channels measures one or more spectral bands of fluorescence and/or reflectance light. This light splitting embodiment is preferably designed to minimize the crosstalk between the imaging channels and maximize the optical throughput.

Preferably, it is possible to miniaturize the imaging device or guide the optical paths through medical devices such as endoscopes, fiberscopes or laparoscopes such that the device and method can be used for minimally invasive interventions, or diagnostic imaging techniques, like e.g. endoscopy, laparoscopy, etc.

A preferred way of correcting marker light images, also referred to as "data correction", is described in more detail in the following.

Correction procedures, which are preferably applied in real-time, may lead to at least partially artifact-free marker images (i.e. fluorescence images) which accurately represent the spatial distribution of fluorophores and chromophores. Several corrected schemes can be responsible for an integrated correction of all effects that may undergo in a real-time measurement, although a selective number of steps may be applied at specific applications.

In the following, a preferred approach of the inversion of the image-blurring effects due to space-variant scattering and absorption (and therefore optical path length) within the biological tissue is described, an effect mainly causing a different distribution of the pixel intensities in the neighborhood of its "correct" appearance. Preferably, contrary to common deconvolution strategies, the Point Spread Function (PSF) is space-variant, and additionally not constant along different measurements - as its parameters originate in the tissue under test itself and are not (only) induced by the imaging system. However, an advantage is, that the spatially varying total point spread function (tpsf) can be measured with sufficient accuracy, as described above.

The inversion of image-folding effect by this tpsf can, therefore, be performed by a generic spatially variant non-blind deconvolution algorithm, as described by I. Terms, "SPATIALLY-VARIANT LUCY-RICHARDSON DECONVOLUTION FOR MULTIVIEW FUSION OF MICROSCOPICAL 3D IMAGES", Institute of Computer Science , Albert-Ludwigs-University of Freiburg , Chair of Pattern Recognition and Image Processing, Georges-K, "BIOSS Centre for Biolog", pp. 899-904, 2011.

To fulfill the real-time requirements of e.g. an intraoperative fluorescence imaging application, a preferred possible advantageous implementation is:

(1) Binning of all occurring tpsf parameters and calculating spatially invariant Lucy-Richardson deconvolutions. By splitting said calculation into several (e.g. i = 20) independent tasks - one for each parameter-bin - the calculation is parallelizable without additional computational efforts.

(2) In the following step, the corrected result image (spatially variant deconvolution) can be integrated in a spatially variant manner, by integrating the corresponding deconvolution results (ith bin) of the previous step on a per-pixel basis depending on its tpsf parameters, and a distance metric according to the tpsf bin result.

The device and method disclosed herein also enable the utilization of other spatially invariant deconvolution concepts which can gain from the knowledge of the correlated space-depending PSFs.

In an advantageous implementation, the combination of a real-time capable, less-accurate implementation can be combined with a longer-time-consuming (several seconds) image-processing method, which offers even improved accuracy.

An improved accurate image processing might then be utilized for important imaging situation during interventions. An example would be the decision whether to excise additional tissue during oncologic surgery.

The abovementioned aspects of generating at least one corrected marker light image (corrected fluorescence image) by deconvolving at least one marker light image (fluorescence image) using at least one spatially varying total point spread function determined from at least one patterned sample light image are preferably based on the approach that a combination of two novel concepts relating to capturing and reverting the effect of optical properties leads to improving fluorescence molecular imaging (FMI) performance. The first concept preferably relates to spatially resolving the effects of tissue on the fluorescence image. The second concept relates to using this spatially dependent information to correct fluorescence images, enabling for the first time the application of deconvolution to FMI. Uniting these two concepts, which is also referred to as "Spatially Adaptive Impulse Response Correction" or "SAIRC", is a novel framework that uses spatially variable kernels in an adaptive fashion to correct the fluorescence images. Advantageously, an inexpensive Digital Light Processing (DLP) projector can be used that excites spatial impulse responses (PSFs) across the field of view imaged by projecting a pattern of illumination points (delta functions) onto tissue. SAIRC captures these spatial impulse responses of tissue and assembles them in a matrix of spatially varying kernels that drives a variant deconvolution scheme. Preferably, a spatially adaptive deconvolution based on a modified fast Lucy-Richardson algorithm is used, as described by Dey, N. et al., "Richardson-Lucy algorithm with total variation regularization for 3D confocal microscope deconvolution", Microsc. Res. Tech. 69, 260-266 (2006).

In the following, preferred embodiments of the device, apparatus and methods and corresponding studies to demonstrate the SAIRC principle in phantoms and animal measurements are described and it will be discussed how SAIRC can be implemented in clinical FMI systems for real-time applications.

The objects, e.g. phantoms and/or tissues, are preferably imaged using the device and workflows described above with reference to Figures 1 to 4. Alternatively or additionally, devices and methods (or aspects thereof) described as follows can also be used.

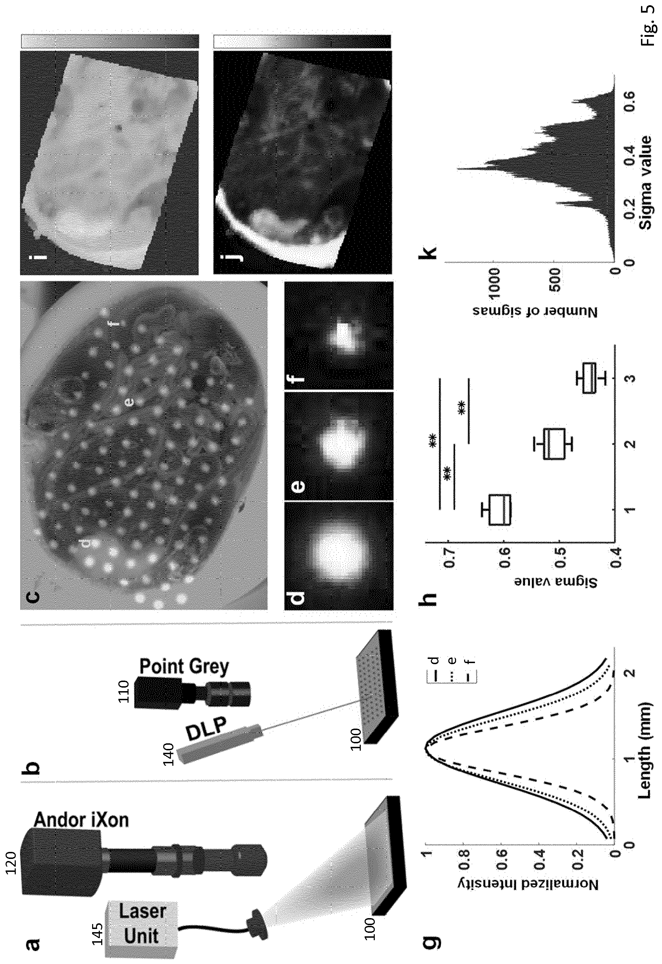

Figure 5 shows a schematic of the SAIRC process to enhance fluorescence imaging: Schematic system configuration for (a) fluorescence image acquisition and (b) scanning with the projector. (c-f) Color image of tissue from a mouse with an HCT116 tumor embedded in O.C.T. (Optimal Cutting Temperature) compound after several cuts, overlaid with some of the acquired impulse response images from the area scanned. The small panels underneath show images of spots from different areas: (d) tumor; (e) intestine; (f) muscle. (g) Comparison of the normalized profile of Gaussian curves fitted to the points d, e, and f. (h) Distribution of the sigma values fitted to five random spots chosen from areas 1, 2, 3 around points d, e, f. (i) Two-dimensional sigma map, extracted from the fitted Gaussians for the whole scanning area. (j) Sum of all the acquired impulse response images. (k) Histogram of sigma values binned into n bins, each of which contained the same number of sigma values.

Figure 5 shows an alternative embodiment of implementing the illumination and image relay of sample light images and marker light images in separate optical paths and/or apparatuses. Nevertheless, regarding the region under investigation 100, excitation illumination 145, fluorescence image sensor 120, illumination device 140 and sample light camera 110 the elucidations set forth above with reference to Figures 1 and 2 apply accordingly. Depending on the requirements of the application, the shared use of relay optics as shown in Figures 1 and 2 and discussed above may be preferred.

Preferably, the objects imaged are illuminated by a spatial impulse response using a DLP projector at 624/18 nm wavelength (Fig. 5b) that was scanned throughout the tissue. The broadening of the impulse responses emitted at tissues was captured by a monochromatic camera (Fig. 5c-f). Different parts of tissue from an HCT116 tumor-bearing mouse demonstrated different broadening, which is characteristic of different optical properties. As an example, highly scattering cancerous tissue (spot d; Fig. 5c) shows higher broadening (Fig. 5d) compared to an impulse response collected from intestinal and muscle tissue (spots d, e, f; Fig. 5d-f). At each of the three spots studied herein, a 2D symmetrical Gaussian function was fitted (Fig. 5g) to better illustrate the differences in broadening and optical properties among the different tissue types.

Preferably, the standard deviation (sigma) is extracted from each fitted Gaussian for random spots (spots d-f; Fig. 5c-f) and for four spots in each of the areas 1 (cancer), 2 (intestinal), and 3 (muscle), where each of the areas was defined as having relatively uniform optical properties. The sigma value distribution (Fig. 5h) showed that sigma varied only slightly within each area, and that the sigma distribution varied significantly across different areas according to the Mann-Whitney test (p value stars in Fig. 5h). After scanning the entire tissue surface with the projected DLP point and extracting the sigma value from each Gaussian fitting (see section "Methods" below for further details), a 2D sigma map (Fig. 5i) was assembled that can be used as a guide for tissue diffusion. The sum of all the projected points (Fig. 5j) resembles the illumination of the tissue with a homogeneous planar source.

Preferably, the sigma map (Fig. 5i) is also employed to assemble the PSFs that are used for variant deconvolution of the blurred fluorescence image of the tissue. From the histogram of the sigma map (Fig. 5k), the sigma values are binned into n equally sized bins, and the lowest value of each bin is used to construct a Gaussian kernel. Preferably, this kernel is deconvolved with the raw blurry fluorescence image using a modified Lucy-Richardson deconvolution scheme. The deconvolution process is repeated for every binned kernel, and the final corrected fluorescence image is preferably constructed using a weighted summation of all the deconvolved images. This binning process minimized the deconvolution time with multiple kernels.

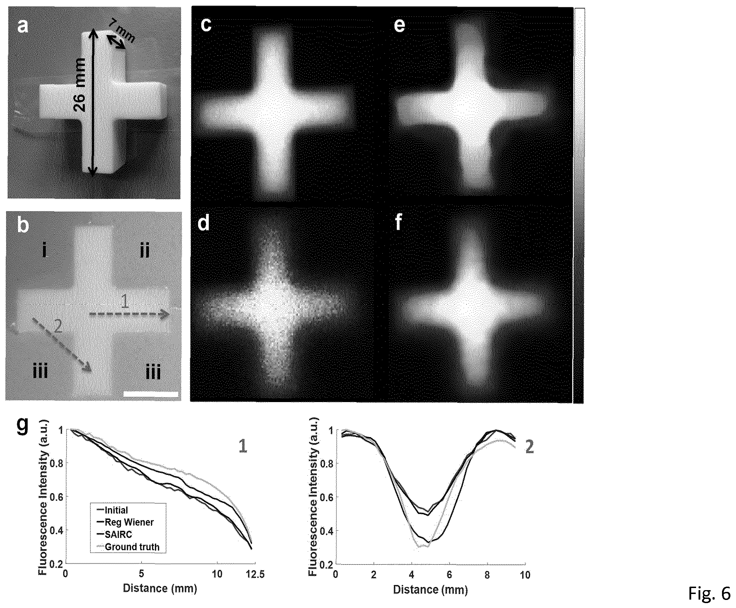

Figure 6 shows an example illustrating the SAIRC improvement in fluorescence image quality of a phantom. (a) Color image of the phantom surrounded by air. (b) Color image of the phantom surrounded by three areas (i, ii, iii) with different optical properties based on different ink concentrations (see main text). Scalebar: 10 mm. (c) Ground truth fluorescence image. (d) Raw fluorescence image of panel (b). (e) Fluorescence image after the implementation of SAIRC. (f) Fluorescence image after the implementation of regularized Wiener deconvolution. (g) Fluorescence intensity profiles 1 and 2 from panel (b). Intensities 1 and 2 are normalized to 0-1. Initial: Raw Fluorescence Image, Reg Wiener: regularized Wiener deconvolution, SAIRC: Deconvolved Image with spatially adaptive kernels.

A phantom can be designed to explore basic parameters of the disclosed SAIRC framework and to determine whether SAIRC can perform well even when the acquired image contains substantial noise as exemplarily shown in Fig. 6. A cross-shaped agar phantom containing Alexa Fluor 750 and dairy cream ($\mu$s = 150 cm-1, $\mu$a= 0.05 cm-1) (Fig. 6a) was embedded into a background agar medium of different optical properties (Fig. 6b). The background medium consists of area i ($\mu$s= 150 cm-1, 2.32 cm-1), area ii ($\mu$s= 150 cm-1, $\mu$a= 1.16 cm-1) and area iii ($\mu$s= 150 cm-1, $\mu$a= 0.58 cm-1), each formulated with different concentrations of black ink (see section "Methods" below for further details). Because of their different optical properties, these areas are assumed to modify the projected points differently.

Fig. 6d shows the raw fluorescence image of the phantom of Fig. 6b. The difference between the edges of area i and the edges of areas ii and iii can be observed: the edges in area i appear more resolved. In contrast, diffusion dominates in area iii, and the phantom's edges appear more blurry. Fig. 6e shows the results of the SAIRC process using the sigma map from Fig. 6c (100 iterations, $\lambda$=0.02), while Fig. 6c is the fluorescence image of the phantom surrounded by air (Fig. 6a). Fig. 6c was considered the ground truth image (see section "Methods" below for further). The results of SAIRC can be compared with those of regularized Wiener deconvolution (see section "Methods" below for further details), which uses regularization and a fixed PSF (Fig. 6f). The SAIRC-corrected image (Fig. 6e) is better resolved than the initial image (Fig. 6d) and the image from the other deconvolution method (Fig. 6f), and it resembles the ground truth image (Fig. 6c) much better. The edges are adequately restored from round to rectangular, recovering the original shape of the phantom; in addition, the noise artifacts are suppressed by the use of the regularization term $\lambda$, and the shape is smoothed out. Preferably, a relatively high $\lambda$ value can be used, reflecting the greater amount of noise in the acquired image.

To quantify the ability of SAIRC to restore image sharpness and edge quality, fluorescence intensity profiles can be drawn (dotted lines 1 and 2 in Fig. 6b). In Fig. 6g the intensity profiles in the raw, SAIRC, regularized Wiener and ground truth images are compared, and it can be observed that the SAIRC profile was closest to the ground truth image for both lines. Furthermore, the image sharpness can be assessed by calculating the Brenner gradient (see section "Methods" below for further details), which was 0.0018 for the initial image, 0.0022 for the SAIRC-corrected image and 0.0010 for the regularized Wiener image. Consequently, the SAIRC-corrected image is 22% sharper than the initial image. The root mean square (RMS) difference from the ground truth image is estimated to be 0.0621 for the initial image, 0.0445 for the SAIRC-corrected image, and 0.0457 for the regularized Wiener corrected image. This indicates that SAIRC increases similarity to the ground truth image by 28% compared to the raw image.

Apart from phantoms, the SAIRC algorithm can be tested by imaging abdominal cryosections of mice bearing 4T1 tumors injected with the tumor-targeting fluorescence agent Integrisense 750, or mice bearing HCT116 tumors expressing the near-infrared fluorescent protein IRFP720. The regularization parameter $\lambda$ was lower for this imaging than for phantom imaging, since noise was lower in the mouse tissue.

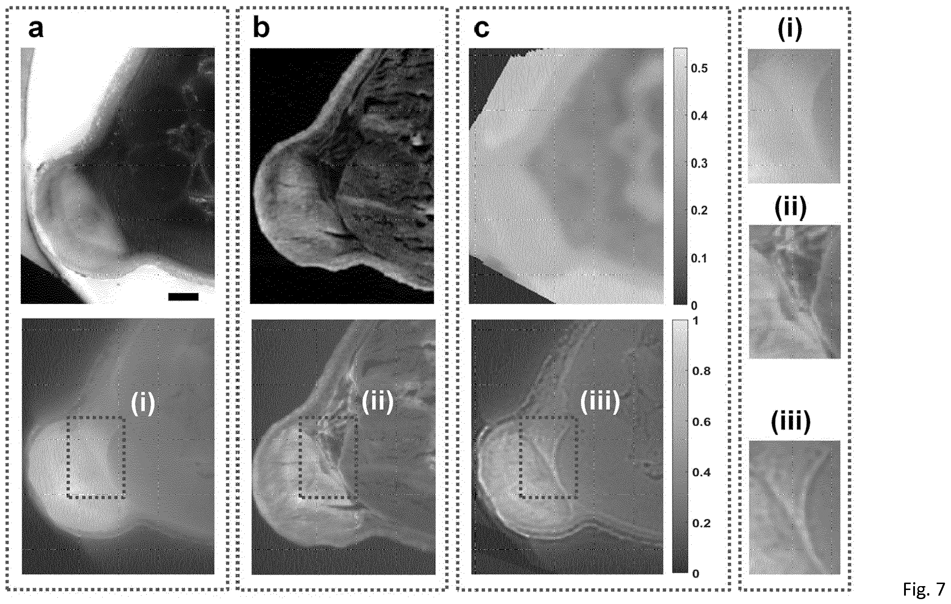

Figure 7 shows examples illustrating the SAIRC improvement in the quality of fluorescence images of one mouse containing a 4T1 tumor injected with Integrisense 750. (a) Upper: color image of the back of a mouse bearing a 4T1 tumor after embedding in OCT compound. Lower: the corresponding raw fluorescence image of the tissue block. Scalebar: 1 mm. (b) Upper: color image of a thin slice (100 $\mu$m) from the OCT-embedded tissue block in front of a black background. Lower: the corresponding fluorescence image of the thin slice, which served as the ground truth image. (c) Upper: sigma map acquired from the scanning process (color bar in mm). Lower: corrected fluorescence image after the implementation of SAIRC. (i-iii): Zoomed-in areas of the regions of interest in the lower images in panels (a), (b) and (c).

Upper panels of Fig. 7a and 7b depict color images of, respectively, the OCT-embedded tissue block and a thin slice from the block. Lower panels of Fig. 7a and 7b show the corresponding raw fluorescence images. The fluorescence image of the thin slice (Fig. 7b) is considered the ground truth, due to its diffusion-free thickness. Comparison of the raw fluorescence image in Fig. 7b with the SAIRC-corrected fluorescence image (lower panel in Fig. 7c) shows that deconvolution mitigated the effect of light diffusion on the image quality, providing higher resolution, sharpness and resemblance to the ground truth image. Comparison of zoomed-in regions i, ii and iii further revealed how SAIRC correction clarified details in the fluorescence image, bringing it closer to the ground truth image. In fact, SAIRC was associated with a doubling of sharpness, as quantified with the Brenner gradient (0.0003 for Fig. 7a vs 0.0006 for Fig. 7c). SAIRC also increased similarity to the ground truth image by 37%, as quantified with RMS (Root Mean Square) (0.1918 for Fig. 7a vs 0.1402 for Fig. 7c). Some discrepancies between the corrected image and the thin slice can be attributed to tissue distortion during the cutting process.

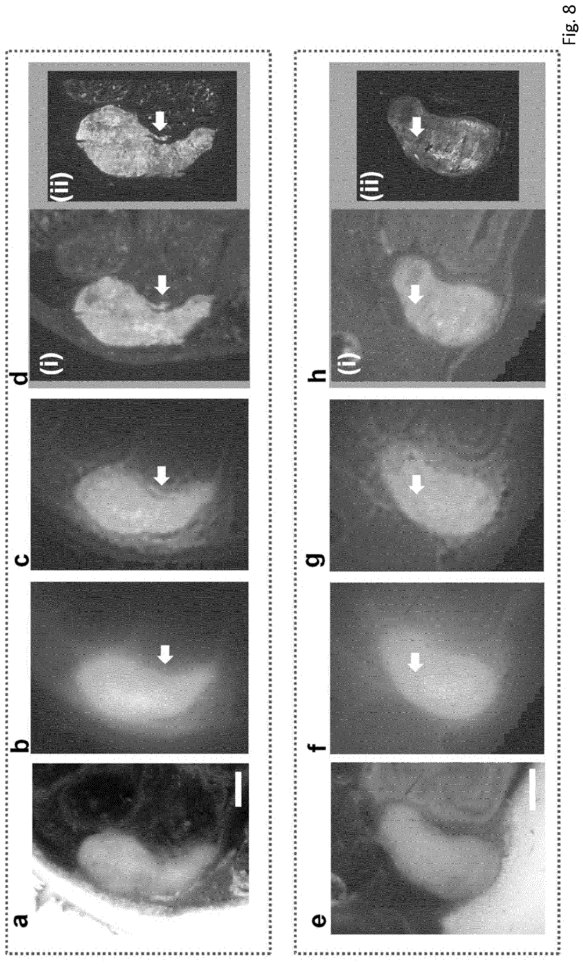

Similar improvements in image quality can be observed when the imaging experiments are applied to tissue from two mice bearing HCT116 tumors expressing IRFP720, which is shown in Figure 8 illustrating SAIRC improvement in quality of fluorescence images of mice with HCT116 tumors expressing IRFP720. The upper row corresponds to one mouse, while the lower row corresponds to a second mouse. (a, e) Reflection images of the abdominal area, after embedding in OCT compound. Scalebar: 1 mm. (b, f) Raw fluorescence image of the tissue block in panels (a) and (e). (c, g) Corrected image after the implementation of SAIRC. (d, h) (i) Fluorescence image of a thin slice (50 $\mu$m) of the tissue block obtained under the same conditions as the block image. (ii) Stitched images of the thin slices from a commercial fluorescence microscope. White arrows indicate tumor features that become clear only after SAIRC correction.

In the case of these two mice, SAIRC correction (Fig. 8c and g) bring the fluorescence images closer to the ground truth image (Fig. 8d (i) and 8h (i)) than the initial images (Fig. 8b and 48f). As an additional verification of the results achieved with the device and method disclosed herein, stitched fluorescence microscope images of the thin slices can be used. The resulting images (Fig. 8d(ii) and 8h (ii)) are consistent with those obtained with the device and method disclosed herein. Some tumor characteristics, which are marked with white arrows, are not visible in the raw fluorescence image but are revealed after SAIRC correction.

As shown above, SAIRC is a novel fluorescence image correction scheme, which accurately captures optical property variations across the whole imaging area and enhances the acquired image via an iterative deconvolution process. A preferably point-like beam from a DLP scans the imaging area, and spatially variant deconvolution kernels are retrieved from every corresponding beam region in the image and fitted to symmetrical Gaussian curves. Then, a Lucy-Richardson deconvolution method is applied, in which a regularization parameter (as described by Dey, N. et al., see above) is used to suppress noise, and kernel spatial variability is binned in order to minimize computational time. The final corrected image is preferably the result of a weighted sum of the deconvolution results with the binned kernels. The efficiency of the device and method can be demonstrated using custom-made phantoms and ex vivo tissues from tumor-bearing mice. SAIRC improved fluorescence image resolution and contrast, increasing resemblance to the ground truth image by up to 37%. This approach may increase the sensitivity and accuracy of FMI in preclinical and clinical applications, including surgical guidance and endoscopy.

According to another preferred aspect of present disclosure, the diffusion modification at each point on the tissue is captured by scanning over the tissue and translating the impulse response from every location to a representative value. This allows for visualizing how diffusion in the tissue modifies the projected kernel in a 2D map. This map can indicate how much more diffusive one tissue area is relative to another. This information helps SAIRC capture the spatial heterogeneity of the tissue and import this spatial variability into a correction method based on deconvolution.

SAIRC correction enhances image quality and structural details in phantoms and tumor tissue in mice. We further shown above, SAIRC can perform well even when the acquired image contains a large amount of noise, such as with a highly noisy phantom. Greater improvements in sharpness and accuracy can be obtained with mouse tissue than with phantoms. This can be attributed to the lower quality of the initial raw phantom image, where noise is greater. As further demonstrated above, SAIRC provides better enhancement than another deconvolution method that uses regularization and invariant PSF.

Preferably, SAIRC has the potential to be used with different combinations of imaging systems, tissue types and near-infrared fluorescence agents. The current method can be used with a variety of fluorescence agents upon proper modification of the projector light source. SAIRC has the potential to be applied in fluorescence surgical navigation, where it can provide better insight into tumor structure and surroundings (Figs. 7-8). This is very important, since more accurate imaging of tumor areas offers better tumor delineation during surgical navigation and better insight into tumor topology. Preferably, SAIRC is implemented in or transferred into clinical FMI environment, offering better tumor delineation and insights into tumor heterogeneity. When implementing SAIRC into clinical FMI, a real time visualization is preferred, including a very fast scanning acquisition of the reflected points, fitting and deconvolution time. In order to make the scanning as fast as possible, scanning at multiple points simultaneously and/or implementing the fitting process for the sigma extraction in a parallel instead of serial regime is preferred. To minimize deconvolution time, a low number of bins and therefore deconvolutions can be used. It is also possible to adopt more sophisticated approaches for the fitting process (e.g. summation of non-symmetrical Gaussians) and more sophisticated schemes for weighing the different deconvolution results.

Methods

a) Phantom

A negative mold of a cross with dimensions 27 x 27 x 6 cm was developed and a mixture of scattering, absorbing, fluorescent agent and base material was cast inside the mold. Agar (1%) was selected as the base material, dairy cream (30% fat) as scattering agent, and acrylic ink as absorbing material. Alexa Fluor 750 (Thermo Fisher Scientific) was used as the fluorescent agent. The final composition of the phantom was 4 nM of Alexa Fluor 750, 15% fat dairy cream and 1% agar (Fig. 6a).

To test the efficacy of the preferred deconvolution method, three additional areas of different optical properties were created around the fluorescence area. The three areas were formed by successive filling, solidifying and removing of excess agar, ink and cream milk in a petri dish. All three areas contained 15% fat dairy cream. Area i also contained 0.25% ink; area ii, 0.125% ink; and area iii, 0.0625% (Fig. 6b).

b) Animal models

All animal procedures were performed in accordance with protocols approved by the Government of Upper Bavaria. The cryo-imaging experiments were performed with female NOD SCID Shorn mice (Envigo, Germany) approximately 8 weeks old. In two mice, 2 x 106 HCT116 human colon cancer cells expressing IRFP720 (ATCC, CCL-247, transfected by us) were injected into the abdominal area. In a third mouse, 1 x 106 4T1 breast cancer cells (ATCC, CRL-2539) were injected into the mammary fat pad. Tumors were allowed to grow until approximately 8 mm. Then the animal was injected intravenously with Integrisense 750 (2 nmol; PerkinElmer), and sacrificed 24 h later. Mice were frozen at -50 oC and cryosliced as described 40,41 for imaging.

c) System and data acquisition

For mouse cryo-imaging, a cryoslicing system equipped with a fluorescence camera (Luca R EMCCD, Andor) was used. For the excitation of HCT116 mice, a 680-nm CW diode laser (B&W Tek, 300 mW) was used; for the 4T1 mouse, a 750-nm CW diode laser (B&W Tek, 300 mW) was used. A 721/42 filter (Chroma) was employed for acquiring fluorescence images of the HCT116 mice and an LP 785 filter (Chroma) was used for the 4T1 mouse. For the fluorescence images that were used as ground truth, thin slices of 50 $\mu$m from the block tissue were imaged using the cryo-imaging system.
Stitched fluorescence microscopy images were acquired using a Zeiss Axio Imager M2 microscope with Axio-oCam MRm monochrome digital camera.

Ground truth images

In the case of phantoms, image blurring was considered to be affected mainly by the optical properties of the three areas surrounding the phantom and not by the fluorescent phantom itself. For this reason, the acquired image of the phantom surrounded by air was considered as the ground truth image and it was averaged to suppress the noise. In mouse tissue experiments, we considered the thin slice to be minimally affected by diffusion and therefore to be the closest to the true fluorescence distribution within the specific slice. Slices of 50 $\mu$m were acquired, so that fluorescence signal would be strong enough.

Retrieval of the kernels

Due to the spatial variability of the convolution kernel and the discrete nature of the image, a K(x,y) kernel function was defined, where x,y are the pixel coordinates, to represent the kernels for every pixel within the captured image. To calculate each single kernel, a point-like source was projected onto the sample surface by means of a DLP projector (DLP 3000 Lightcrafter, Texas Instruments, Dallas, USA), and the reflection image produced was recorded. Each reflection image represents the spatially-resolved reflectance of the source and it depends on the optical properties of the sample at this specific point. This image was considered as an approximation for the unknown convolution kernel that essentially blurs the true fluorescence image locally. This point-like source was used to scan the entire area of interest, and all reflection images were recorded for the whole area. As a result, a unique kernel was assigned to each image pixel.
The projector is preferably positioned above the sample at a very small angle (below 5 degrees) such that the projected spots do not appear distorted. Only the red LED channel (624/18 nm) of the projector was utilized since it was the closest to the absorbance and emission peaks of the fluorescence agents in the phantom and mouse tissue. Instead of using the raw reflection image as a kernel, every reflection point image was fitted to a symmetrical, two-dimensional Gaussian curve, although more complex models involving sums of Gaussians exist. Each Gaussian curve was considered homogeneous in all directions and the standard deviation value (sigma) was retrieved for every curve.
In order to retrieve values from the whole imaging surface, the latter was scanned by the point-like beam source on a pixel-by-pixel basis. After Gaussian fitting and averaging of the neighboring pixels, a two-dimensional array of sigma values was formed, containing a sigma value for every pixel of the acquired raw fluorescence image. The reflection images were recorded by means of a second camera, but it is also possible to use the fluorescence imaging camera without any emission filter. Sigma values were adjusted using the same calibration surface for the various experiments, and all the images were co-registered.
To correct for uneven light projection, the reflection image was divided by the reflection image of a homogeneous white surface when all the pixels of the projector were on. To disengage the projected spot from the projection system characteristics, the following is preferred: It is considered that the measured kernel ($K_{measured}$) equals the convolution (eq. 2) of the raw trace ($I_{projector}$) on the imaging surface with the unknown kernel (K) to be resolved. It is assumed that kernel used in the deconvolution scheme (K)xxxxx depends exclusively on the local optical properties of the tissue.

$$K_{measured} \approx I_{projector} * K \quad (2)$$

In order to estimate the spatially variable $I_{projector}$, preferably a homogeneous black semi-reflective surface is used and the aforementioned scanning procedure is repeated to retrieve reflectance images that approximate the $I_{projector}$. Symmetrical Gaussian curves are then fitted in an effective way (coefficient of determination > 0.85), and a two-dimensional array with sigma values is generated. Finally, since $K_{measured}$ and $I_{projector}$ were both approximated by Gaussians, it is possible to extract the sigma of the kernel K from the following equation (eq. 3), which is based on Gaussian convolution properties:

$$sigma_K = \sqrt{sigma_{K_{measured}}^2 - sigma_{I_{projector}}^2} \quad (3)$$

d) Data Processing

Deconvolution

The raw acquired image is considered to be the result of a convolution operation of the true fluorescence image with a kernel that is dependent upon the optical properties of the imaging area. Preferably, the distortion introduced by the optical imaging system is considered negligible compared to diffusion effects due to light-tissue interactions.

After assigning a sigma value to every pixel, deconvolution for each pixel is performed separately. Nevertheless, a per-pixel deconvolution step would be extremely computationally intensive and, thus, time-consuming. Therefore, it is preferred that the sigma values are binned into n equally sized bins and the lowest sigma value of every bin is used to create a new Gaussian kernel which is taken as the input kernel for the upcoming deconvolution step. For the deconvolution step, it is preferred to perform a Lucy-Richardson iterative restoration method. Further, a regularization constraint based on total variation is used to suppress the noise that is amplified during the iterations. Finally, m cycles of deconvolution are performed and the results are weighted according to eq. 4:

$$W_i = 1 - \left( \frac{|\sigma(x, y) - \sigma_{bin(i)}|}{\max(\sigma) - \min(\sigma)} \right) \quad (4)$$

where $W_i$ is the weight for the ith deconvolution of the (x, y) pixel, $\sigma(x,y)$ is the corresponding sigma value from the sigma map and $\sigma_{bin(i)}$ is the binned sigma value that was used for the construction of the kernel of the ith deconvolution. The contribution of each deconvolution result to a given pixel is higher when the sigma value of that pixel is closer to the sigma value of the kernel used to produce the deconvolution result.

Evaluation of performance

The sharpness of the corrected fluorescence images is preferably examied using the Brenner gradient, which is an image differentiation function that can be used as a measure of image focus. The larger the gradient, the sharper the image is. Furthermore, the root mean square (RMS) of the difference between corrected and ground truth images was used as a metric to assess improvement due to the correction process. The lower the value, the more the corrected image resembles the ground truth.

Finally, SAIRC can be compared with another deconvolution method that uses regularization and a fixed PSF. This method is based on regularized Wiener deconvolution and is implemented in the ImageJ plugin FFTJ. For the PSF, a homogeneous Gaussian kernel is simulated based on the average value from the sigma map. The regularization parameter was optimized by visual inspection in order to reach a compromise between blurriness and appearance of artifacts in the final image.

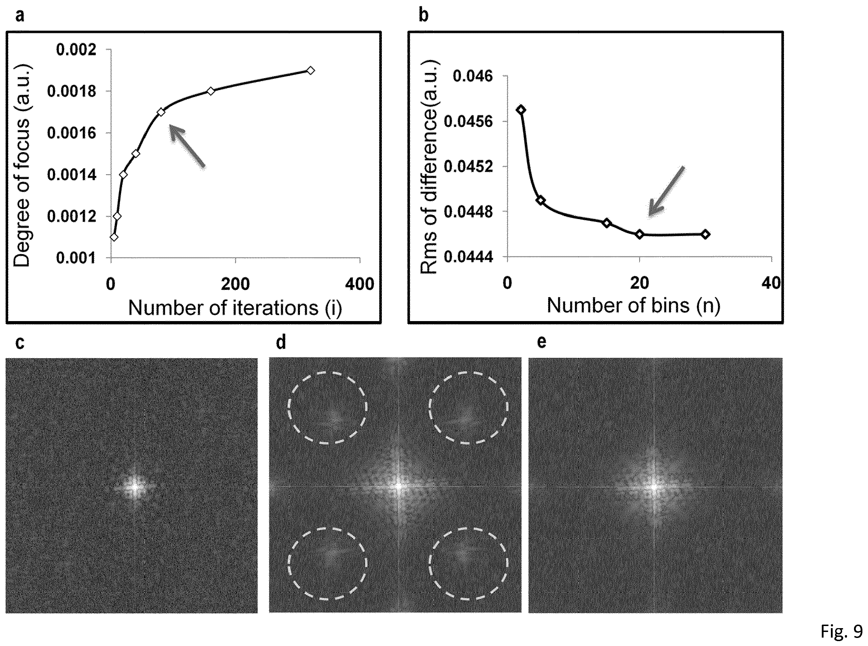

Figure 9 illustrates metrics to estimate optimal parameters for the deconvolution algorithm. (a) Evaluation of image sharpness regarding the number of iterations. (b) Standard deviation of the image difference regarding the number of the sigma bins. (c) Amplitude Fourier domain of the initial fluorescence image. (d) Amplitude Fourier domain of the deconvolved fluorescence image (high λ), (e) Amplitude Fourier domain of a deconvolved fluorescence image (suitable λ).

Estimation of deconvolution parameters

In order to optimize the number of iterations, the number of bins, and the regularization parameter ($\lambda$), all of which are inputs in the deconvolution algorithm, tests are performed with different parameter combinations and the values that give the best performance according to quality metrics are identified. The optimal number of iterations can be tested by assessing the image sharpness with respect to different iteration numbers. Fig. 9a depicts the Brenner's degree of focus measure regarding the number of iterations with a certain number of bins and $\lambda$ (20 bins, $\lambda = 0.02$). A trend of increasing Brenner's gradient was observed as the iterations increased between 5 and 100. Beyond 100 iterations, the increase in sharpness slowed. Even if the degree of focus was slightly better after 320 iterations, some artifacts may start to appear in the image. For this reason, it is preferred to perform < 100 iterations to keep computation time ~ 1 min without compromising image quality.

To select an appropriate amount of bins for the sigma values, the RMS of the difference between the corrected and ground truth fluorescence images is used as a metric. As observed in Fig. 9b, for a certain number of iterations and $\lambda$ (iterations=100, $\lambda$=0.02), the RMS seemed to decrease as we increased the number of bins, as expected. After 20 bins, though, the RMS seemed to start saturating, indicating that a large number of bins would not improve the quality of the deconvolution result. Thus, the computational time can be minimized by using preferably only 20 bins.

The regularization parameter $\lambda$ should preferably be selected in a way that is high enough to suppress the image noise but low enough so it will not impede the restoration process. For high $\lambda$, points of very high intensity become more prominent because they are amplified at each iteration. This can be noticed in the Fourier amplitude space of the image. Fig. 9c represents the amplitude Fourier space of the raw blurred fluorescence image, while Fig. 9d represents the equivalent for the deconvolved image. It is observed that more frequency content was restored after deconvolution, as expected. Nevertheless, the amplification of other parasitic frequencies may also be observed (dotted circles in Fig. 9d) as $\lambda$ increases. For this reason, after inspecting the Fourier space of the deconvolved image, the maximum $\lambda$ is selected preferably just before the appearance of the parasitic frequencies. For the phantom, $\lambda$ values around 0.02 are selected, whereas for the tissue values around 0.001. The higher value of the phantom parameter can be attributed to the much greater noise in the phantom than in mouse tissue.

## Claims

1. A device for imaging an object, in particular a biological tissue, the device comprising

   - at least one illumination unit (140, 145, 410) configured to illuminate a region of interest (100) of an object with homogeneous illumination light configured to stimulate the region of interest (100) of the object to emit emission light, in particular fluorescence light, and
   patterned illumination light exhibiting a spatially varying light intensity over the region of interest (100) of the object,
   - at least one image capturing unit (110, 120, 420) configured to capture at least one marker light image (432) by detecting the emission light emitted by the region of interest (100) and
   at least one sample light image (431) by detecting light reflected, in particular diffusely reflected, by the region of interest (100) upon illumination with the patterned illumination light, and
   - a processing unit (440, 450) configured to generate at least one corrected marker light image (460) based on the at least one marker light image (432) and the at least one sample light image (431).

2. The device according to claim 1, the processing unit (440, 450) being further configured to

   - extract optical property information regarding at least one optical property, in particular light absorption and/or light scattering coefficients, of the object from the at least one sample light image (431) and
   - generate the at least one corrected marker light image (460) by correcting the at least one marker light image (432) using the optical property information.

3. The device according to claim 2, the processing unit (440, 450) being further configured to

   - determine at least one spatially varying total point spread function (tpsf) from the at least one sample light image (431) and
   - determine the optical property information from one or more properties, in particular functional parameters, of the total point spread function (tpsf) determined from the at least one sample light image (431).

4. The device according to claim 1 or 2, the processing unit (440, 450) being further configured to

- determine at least one spatially varying total point spread function (tpsf) from the at least one sample light image (431) and
- generate the at least one corrected marker light image (460) by deconvolving the at least one marker light image (432) using the at least one spatially varying total point spread function (tpsf) determined from the at least one sample light image (431).

5. The device according to claim 3 or 4, the processing unit (440, 450) being further configured to determine the at least one spatially varying total point spread function (tpsf) by fitting at least one function, in particular a Gaussian function and/or a model function describing a modification of a point illumination by absorption and scattering in diffusive media, to the at least one sample light image (431).

6. The device according to any preceding claim,

- the homogeneous illumination light exhibiting a spatially homogeneous light distribution over the region of interest (100) and/or a constant intensity over the region of interest (100) and/or
- the patterned illumination light exhibiting a spatially inhomogeneous light distribution over the region of interest (100) and/or a two-dimensional distribution, in particular a grid, of points of light.

7. The device according to any preceding claim, the at least one illumination unit (140, 145, 410) comprising a light source configured to emit light and a light modulator configured to spatially modulate the intensity of the light emitted by the light source so as to obtain the patterned illumination light.

8. The device according to claim 7, the light modulator comprising

- one or more light reflecting elements configured to spatially selectively reflect the light emitted by the light source so as to obtain the patterned illumination light and/or
- one or more light transmitting elements configured to spatially selectively transmit the light emitted by the light source so as to obtain the patterned illumination light.

9. The device according any preceding claim, the at least one image capturing unit (110, 120, 420) comprising

- a marker light image camera (120) configured to detect the emission light emitted by the region of interest (100) of the object,
- a sample light image camera (110) configured to detect the light reflected by the region of interest (100) of the object upon illumination with the patterned illumination light, and
- a light splitting optics (130, 131), in particular a beam splitter or dichroic mirror, configured to relay the emission light emitted by the region of interest (100) of the object to the marker light image camera (120) and to relay the light reflected by the region of interest (100) of the object upon illumination with the patterned illumination light to the sample light image camera (110).

10. The device according to claim 9, the light splitting optics (130, 131) being further configured to relay the patterned illumination light to the region of interest (100) so as to illuminate the region of interest (100) of an object.

11. A method for imaging an object, in particular a biological tissue, the method comprising the following steps:

- illuminating a region of interest (100) of an object with homogeneous illumination light to stimulate the region of interest (100) of the object to emit emission light, in particular fluorescence light,
- illuminating the region of interest (100) of the object with patterned illumination light exhibiting a spatially varying light intensity over the region of interest (100),
- capturing at least one marker light image (432) by detecting the emission light emitted by the region of interest of the object (100),
- capturing at least one sample light image (431) by detecting light reflected, in particular diffusely reflected, by the region of interest (100) upon illumination with the patterned illumination light, and
- generating at least one corrected marker light image (460) based on the at least one marker light image (432) and the at least one sample light image (431).

12. An apparatus for determining and visualizing optical properties of biological tissue, in particular for use in a device and/or method according to any preceding claim, the apparatus comprising

- an illumination unit (140, 410) configured to illuminate a region of interest (100) of an object, in particular a biological tissue, with patterned illumination light exhibiting a spatially varying light intensity over the region of interest (100),
- an image capturing unit (110) configured to capture at least one sample light image (431) of the region of interest (100) by detecting light reflected, in particular diffusely reflected, by the region of interest (100) of the object upon illumination with the patterned illumination light, and
- a processing unit (440) configured to generate, based on the at least one sample light image (431), at least one image visualizing the at least one optical property of the object, in particular light absorption and/or light scattering coefficients.

13. An apparatus for determining correction data, in particular for use in a device according to claim 1 to 10 and/or in a method according to claim 11, the apparatus comprising

- an illumination unit (140, 410) configured to illuminate a region of interest (100) of an object, in particular a biological tissue, with patterned illumination light exhibiting a spatially varying light intensity over the region of interest (100),
- an image capturing unit (110) configured to capture at least one sample light image (431) of the region of interest (100) by detecting light reflected, in particular diffusely reflected, by the region of interest (100) of the object upon illumination with the patterned illumination light, and
- a processing unit (440) configured to generate, based on the at least one sample light image (431), correction data for correcting at least one marker light image (432), which is obtainable by detecting emission light emitted by the region of interest (100) upon illumination with homogeneous illumination light, the correction data relating to

a) at least one optical property of the object, in particular light absorption and/or light scattering coefficients, and/or
b) at least one spatially varying total point spread function (tpsf).

Fig. 1

Fig. 2

Fig. 3

Fig. 4

EP 3 824 799 A1

Fig. 5

Fig. 6

EP 3 824 799 A1

Fig. 7

EP 3 824 799 A1

Fig. 8

Fig. 9

Europäisches
Patentamt

European
Patent Office

Office européen
des brevets

# EUROPEAN SEARCH REPORT

Application Number

EP 19 21 0464

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 2 359 745 A1 (HELMHOLTZ ZENTRUM MUENCHEN [DE]) 24 August 2011 (2011-08-24) | 1,2,7-13 | INV. A61B5/00 A61B1/00 A61B1/04 G01N21/47 |
| Y | * abstract * * paragraphs [0019], [0040] - [0043], [0045], [0050], [0053], [0059], [0065] * | 6 | |
| X | JUNHONG MIN ET AL: "Fluorescent microscopy beyond diffraction limits using speckle illumination and joint support recovery", SCIENTIFIC REPORTS, vol. 3, no. 1, 25 June 2013 (2013-06-25), XP055648088, DOI: 10.1038/srep02075 * abstract * * page 2, right-hand column, line 16 - page 3, left-hand column, line 30 * | 1-5,9-13 | |
| X | TEMERINAC-OTT M ET AL: "Multiview Deblurring for 3-D Images from Light-Sheet-Based Fluorescence Microscopy", IEEE TRANSACTIONS ON IMAGE PROCESSING, IEEE SERVICE CENTER, PISCATAWAY, NJ, US, vol. 21, no. 4, 1 April 2012 (2012-04-01), pages 1863-1873, XP011492023, ISSN: 1057-7149, DOI: 10.1109/TIP.2011.2181528 * abstract * * page 1864, left-hand column, line 24 - page 1867, left-hand column, line 26 * | 1-5, 11-13 | TECHNICAL FIELDS SEARCHED (IPC) A61B G01N |
| Y | US 2012/128264 A1 (YAZDANFAR SIAVASH [US] ET AL) 24 May 2012 (2012-05-24) * paragraph [0053] * | 6 | |

The present search report has been drawn up for all claims

| Place of search | Date of completion of the search | Examiner |
|---|---|---|
| The Hague | 13 May 2020 | Kowalczyk, Szczepan |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 19 21 0464

This annex lists the patent family members relating to the patent documents cited in the above-mentioned European search report.
The members are as contained in the European Patent Office EDP file on
The European Patent Office is in no way liable for these particulars which are merely given for the purpose of information.

13-05-2020

| Patent document cited in search report | | Publication date | Patent family member(s) | | Publication date |
|---|---|---|---|---|---|
| EP 2359745 | A1 | 24-08-2011 | CA | 2789051 A1 | 18-08-2011 |
| | | | CN | 102892348 A | 23-01-2013 |
| | | | EP | 2359745 A1 | 24-08-2011 |
| | | | EP | 2533682 A1 | 19-12-2012 |
| | | | JP | 5986670 B2 | 06-09-2016 |
| | | | JP | 2013519867 A | 30-05-2013 |
| | | | JP | 2015232574 A | 24-12-2015 |
| | | | US | 2013041267 A1 | 14-02-2013 |
| | | | US | 2018177399 A1 | 28-06-2018 |
| | | | WO | 2011098101 A1 | 18-08-2011 |
| US 2012128264 | A1 | 24-05-2012 | US | 2012128264 A1 | 24-05-2012 |
| | | | WO | 2012069496 A1 | 31-05-2012 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- SPATIALLY-VARIANT LUCY-RICHARDSON DECONVOLUTION FOR MULTIVIEW FUSION OF MI-CROSCOPICAL 3D IMAGES. **I. TERMS.** Chair of Pattern Recognition and Image Processing, Georges-K, ''BIOSS Centre for Biolog. Institute of Computer Science , Albert-Ludwigs-University of Freiburg, 2011, 899-904

- **DEY, N. et al.** Richardson-Lucy algorithm with total variation regularization for 3D confocal microscope deconvolution. *Microsc. Res. Tech.,* 2006, vol. 69, 260-266